# EUROPEAN PATENT SPECIFICATION

(11) **EP 1 894 576 B1**
(45) Date of publication and mention of the grant of the patent: **25.05.2011**
(21) Application number: 06756590.3
(22) Date of filing: 25.05.2006
(51) Int. Cl.: A61K 45/06, A61K 31/4704, A61K 31/4709, A61P 3/06, A61P 3/10, A61P 7/02, A61P 9/10, A61P 9/12

(54) **NOVEL TRIGLYCERIDE REDUCING AGENT**
NEUES TRIGLYCERID-REDUKTIONSMITTEL
NOUVEL AGENT RÉDUCTEUR DE TRIGLYCÉRIDE

(30) Priority: 08.06.2005 US 688379 P
(43) Date of publication of application: 05.03.2008
(73) Proprietor: Kowa Company, Ltd., Nagoya-shi, Aichi-ken 460-8625 (JP)
(72) Inventor: YOKOYAMA, Toru, 1670033 (JP); AOKI, Taro, 3590037 (JP)
(74) Representative: Albrecht, Thomas
(86) International application number: PCT/JP2006/310440
(87) International publication number: WO 2006/132091

(56) References cited:
- WO-A1-03/103640
- JP-A- 09 157 258
- JP-A- 2005 504 113
- SAITO Y ET AL: "A randomized, double-blind trial comparing the efficacy and safety of pitavastatin versus pravastatin in patients with primary hypercholesterolemia" ATHEROSCLEROSIS, ELSEVIER IRELAND LTD, IE, vol. 162, 1 January 2002 (2002-01-01), pages 373-379, XP003011498 ISSN: 0021-9150
- JIHONG HAN ET AL: "Pitavastatin Downregulates Expression of the Macrophage Type B Scavenger Receptor, CD36" CIRCULATION 17 FEB 2004, vol. 109, no. 6, 17 February 2004 (2004-02-17), pages 790-796, XP002572025 ISSN: 1524-4539
- ENRIQUE Z. FISMAN ET AL: "Statin research unfinished saga: desirability versus feasibility" CARDIOVASCULAR DIABETOLOGY, vol. 4, no. 8, 7 June 2005 (2005-06-07), pages 1-6, XP002572026 ISSN: 1475-2840
- TAKESHI TANI ET AL: "Cilostazol, a selective type III phosphodiesterase inhibitor, decreases triglyceride and increases HDL cholesterol levels by increasing lipoprotein lipase activity in rats" ATHEROSCLEROSIS, vol. 152, no. 2, October 2000 (2000-10), pages 299-305, XP002572028 ISSN: 0021-9150
- LIU Y ET AL: "Cilostazol (Pletal): A dual inhibitor of cyclic nucleotide phosphodiesterase type 3 and adenosine uptake" CARDIOVASCULAR DRUG REVIEWS, NEVA PRESS, BRANFORD, CT, US, vol. 19, no. 4, 1 January 2001 (2001-01-01), pages 369-386, XP002973725 ISSN: 0897-5957
- MAMI IKENOYA: "Investigation of binding proteins for anti-platelet agent K-134 by Drug-Western method" BIOCHEMICAL AND BIOPHYSICAL RESEARCH COMMUNICATIONS 23 FEB 2007, vol. 353, no. 4, 23 February 2007 (2007-02-23), pages 1111-1114, XP5771111 ISSN: 0006-291X
- DATABASE WPI Week 200808 Thomson Scientific, London, GB; AN 2008-B21012 XP002572030 -& JP 2007 230883 A (KOWA CO LTD) 13 September 2007 (2007-09-13)
- STEVE L. BRAMER ET AL: "Effect of multiple cilostazol doses on single dose lovastatin pharmacokinetics in healthy volunteers" CLINICAL PHARMACOKINETICS, vol. 37 Suppl 2, 1999, pages 69-77, XP009130628 ISSN: 0312-5963
- YAMAMOTO K. ET AL.: 'Effect of NK-104, a new synthetic HMG-CoA reductase inhibitor, on triglyceride secretion and fatty acid oxidation in rat liver' LIFE SCIENCES vol. 65, no. 14, 1999, pages 1493 - 1502, XP003002784
- CROUSE J.R. ET AL.: 'Clinical manifestation of atherosclerotic peripheral arterial disease and the role of cilostazol in treatment of intermittent claudication' JOURNAL OF CLINICAL PHARMACOLOGY vol. 42, no. 12, 2002, pages 1291 - 1298, XP003002785

## Description

### Technical Field

The present invention relates to a hypotriglyceridemic agent containing an HMG-CoA reductase inhibitor selected from the group, consisting of pravastatin, simvastatin, fluvastatin, cerivastatin, atorvastatin, rosuvastatin and pitavastatin and a PDE3 inhibitor as active ingredients.

### Background of the Invention

The statins (HMG-CoA reductase inhibitors) are administrated to hypercholesterolemic patients being expected its high hypocholesterolemic effect. Hypotriglyceridemic effect of the statins are known to be incidental, mild (CE Rackley, Clin.Cardiol. 1996;19(9):683-9, etc), and insufficient; therefore, combined administration with a hypotriglyceridemic agent has been suggested. Ideally, the statins should be administered in combination with the fibrates which have a strong hypotriglyceridemic effect; however, statin-fibrate combination administration causes side-effects for example an increased risk of rhabdomyolysis mainly in patients with kidney damage. Accordingly, the combination of statins and fibrates must be administered cautiously.

Phosphodiesterase (PDE) is an enzyme which hydrolyzes phosphodiester bonds in cyclic nucleotides. To date, 11 isotypes have been identified. PDE3 is one of the isotypes of PDE, inhibited by cyclic guanosine monophosphate (cGMP) and expressed in tissue cells of such as the heart and bronchial tube, adipose cells, platelets, etc. Consequently, PDE3 inhibitors have been used as cardiotonic agents and platelet aggregation inhibitors.

For PDE3 inhibitors, the hypotriglyceridemic effect is reported in a diabetic rat when cilostazol is administered (conference presentation: UEHARA, Kenji et al. Japan Atherosclerosis Society, winter convention (1992) Abstract, Atherosclerosis 1992,20:824) while hypotriglyceridemic effect is unstable in a normal rat (Ministry of Health, Labor and Welfare, 2003, Abstract of application from Otsuka Pharmaceutical Co., Ltd. relating to amplification of efficacy of cilostazol in prevention of relapse of brain infarction). According to clinical report, TG level is lowered after 4-week administration in a subject (literature: MUKOHARA, Nobuhiko et al. Current Pharmacy; 1990,8:618) or return to former value after transient elevation in 24-week administration (literature: DODO Shuji et al. Journal of New Remedies & Clinics. 1996, 45:1837). Also, it has been revealed that levels of free fatty acids are elevated by degradation of TG when milrinone is administered intravenously to a rat (literature: P Cheung et al., Metabolism 2003,52:1496-50). As above, however hypotriglyceridemic action has been reported, distinct effect is not revealed.

The main effect of PDE3 inhibitors is to elevate cyclicAMP (cAMP) levels by inhibiting enzymatic activity of PDE, phosphodiesterases. It has been known for more than 30 years that cAMP that is elevated by administrating PDE3 inhibitor induces/activates lipoprotein lipases. It is also known that cAMP and stable derivatives thereof, cAMP synthetase activating agents (adenylate cyclase) and the PDE inhibitors have the lipolytic effects (literature: D. Baum et al., Proc. Soc. Exp. Biol. Med. 1976, 151:244-8, and many). In this case, it is known that strong lipolytic effects are shown in an *in vitro* experiment with adipose tissues and cells; however, the lipolytic effects are not significant in an *in vivo* experiment with animals.

Generally, many of hyperlipidemic patients exhibit symptoms of hypertriglyceridemia. Thus development of an agent for treating hyperlipemia has been desired, the agent is diminishing the side-effects by combined administration of HMG-CoA reductase inhibitor and inducing effective hypotriglyceridemic effect.

### Disclosure of the Invention

After considerable effort invested in the study, the present inventors surprisingly discovered that combination administration of HMG-CoA reductase inhibitor and PDE3 inhibitor showed significant hypotriglyceridemic effect in the blood, and completed the present invention.

Accordingly, the present invention relates to a pharmaceutical composition comprising an HMG-CoA reductase inhibitor selected among those listed in claim 1 and a PDE3 inhibitor, and a pharmaceutically acceptable carrier, in more detail a pharmaceutical composition for lowering blood triglyceride (TG). The present invention also provides a pharmaceutical composition comprising an HMG-CoA reductase inhibitor selected among those selected in claim 1 and a PDE3 inhibitor, and a pharmaceutically acceptable carrier, wherein the said HMG-CoA reductase inhibtor and PDE3 inhibitor as the active ingredient in a single formulation is administered to a patient in need thereof, in more detail the present invention provides a pharmaceutical composition for lowering blood triglyceride (TG). The present invention further provides a pharmaceutical composition comprising an effective amount of an HMG-CoA reductase inhibitor selected among those listed in claim 1 and a PDE3 inhibitor, and a pharmaceutically acceptable carrier, wherein the composition is administered concurrently or separately at intervals to a patient in need thereof, in more detail the present invention provides a pharmaceutical composition for lowering blood triglyceride (TG).

The present invention also relates to hypotriglyceridemic agent comprising HMG-CoA reductase inhibitor selected among those listed in claim 1 and PDE3 inhibitor. The present invention also provides a hypotriglyceridemic agent comprising an HMG-CoA reductase inhibitor selected among those listed in claim 1 and a PDE3 inhibitor, and a pharmaceutically acceptable carrier, wherein the said HMG-CoA reductase inhibitor and PDE3 inhibitor as the active ingredient in a single formulation are administered to a patient in need thereof. The present invention further provides a hypotriglyceridemic agent comprising an effective amount of an HMG-CoA reductase inhibitor selected among those listed in claim 1 and a PDE3 inhibitor, and a pharmaceutically acceptable carrier, wherein the agent is administered concurrently or separately at a interval to a patient in need thereof.

The present invention also provides a method for lowering the amount of blood triglyceride (TG) or preventing the elevation of the amount of blood triglyceride (TG), which is characterized by administering an effective amount of an HMG-CoA reductase inhibitor selected among those listed in claim 1 and a PDE3 inhibitor concurrently or separately at an interval to a patient in need thereof. The present invention also relates to a composition as described in claim 1 for use in a method for lowering the amount of blood triglyceride (TG) or preventing the elevation of the amount of blood triglyceride (TG), which is characterized by administering an effective amount of an HMG-CoA reductase inhibitor and a PDE3 inhibitor in a single formulation comprising the said HMG-CoA reductase inhibitor and PDE3 inhibitor as the active ingredient to a patient in need thereof.

The present invention also provides use of HMG-CoA reductase inhibitors and PDE3 inhibitors in producing a pharmaceutical composition for lowering blood triglyceride (TG) comprising an effective amount of an HMG-CoA reductase inhibitor selected among the ones listed in claim 1 and a PDE3 inhibitor. The present invention provides use of HMG-CoA reductase inhibitors selected among the ones listed in claim 1 and PDE3 inhibitors in producing hypotriglyceridemic agent, wherein an effective amount of HMG-CoA reductase inhibitor and PDE3 inhibitor is administered in a single formulation. The present invention also provides use of HMG-CoA reductase inhibitors selected among the ones listed in claim 1 and PDE3 inhibitors in producing hypotriglyceridemic agent, wherein effective amounts of HMG-CoA reductase inhibitor and PDE3 inhibitor are administered concurrently or separately at an interval.

The present invention further relates to a composition as defined in claim 1 for use in a method for preventing or treating hypertriglyceridemia, which is characterized by administering effective amounts of HMG-CoA reductase inhibitors and PDE3 inhibitors concurrently or separately at an interval to a patient in need thereof. The present invention also relates to such composition for use in a method for preventing or treating hypertriglyceridemia, characterized by administering an effective amount of HMG-CoA reductase inhibitor selected among the ones listed in claim 1 and PDE3 inhibitor in a single formulation comprising the said HMG-CoA reductase inhibitor and PDE3 inhibitor as the active ingredient to a patient in need thereof.

### Brief Description of the Figures

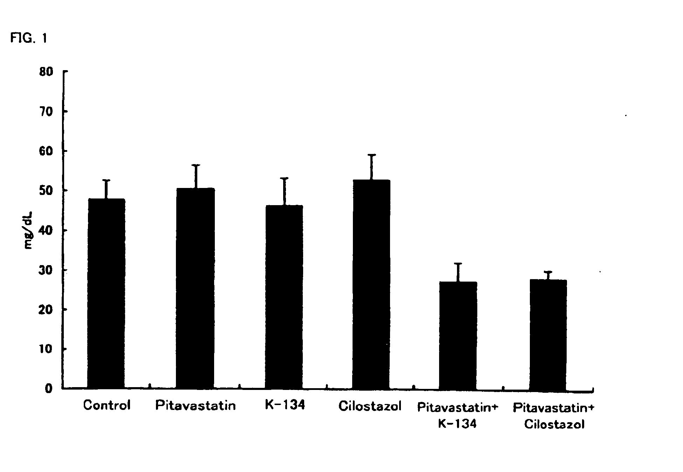

Figure 1. Effect of combined administration of pitavastatin calcium (referred to as pitavastatin) and K-134 or cilostazol on the concentration of plasma triglyceride.

### Best Mode for Carrying out the Invention

The present inventors have tried the various combinations of medical agents in order to improve hypotriglyceridemic effect of HMG-CoA reductase inhibitors. As a result of studying hypotriglyceridemic effects of various combinations of medical agents, the present inventors found surprisingly that the combination of an HMG-CoA reductase inhibitor and a PDE3 inhibitor exhibited outstanding hypotriglyceridemic effect. Although the lipolytic effect of the PDE inhibitors has been reported, the efficacy *in vivo* is not evident, and particularly from consideration of the fact that hypotriglyceridemic effect of PDE 3 inhibitors had not been revealed, the discovery is so remarkable.

The present inventors tested the effect of combined administration of the invention using pitavastatin calcium as an HMG-CoA reductase inhibitor, and K-134 or cilostazol as a PDE3 inhibitor.
To test effects of combination administration of the said agents, rats were used for the experiment. The results are shown in figure 1. Figure 1 shows measurements of blood triglyceride (TG) in rats, wherein the agent was orally administered repeatedly to a rat for 14 days, the rat was fasted for 22 hours after the final administration in the afternoon, and blood sample was collected. The vertical axis in figure 1 indicates concentration of plasma triglyceride (TG) (mg/dL). Administration groups: control; pitavastatin calcium (referred to as pitavastatin) alone; K-134 alone; cilostazol alone; combination of pitavastatin calcium (referred to as pitavastatin) and K-134; and combination of pitavastatin calcium (referred to as pitavastatin) and cilostazol, are listed from left to right.

Not surprisingly, the results show either K-134 or cilostazol, (both PDE3 inhibitors) has little hypotriglyceridemic effect *in vivo*, and it was also indicated that hypotriglyceridemic effects of pitavastatin (HMG-CoA reductase inhibitor) is extremely weak. However, despite that each of the agents shows little hypotriglyceridemic effect, combined administration of these agents showed significant hypotriglyceridemic effect of about 30-40% under fasting.

PDE3 inhibitors for combined administration of the present invention can be PDE3 activation inhibitor effective *in vivo* specifically, for example, enoximone, imazodan, piroximone, isomazole, lixazinone, indolidan, cilostazol, K-134((-)-6-[3[3-cyclopropyl-3-[(1R,2R)-2-hydroxycyclohexyl]ureido]-propoxy]-2-(1H)-quinoline) (Japanese Patent No. 2964029; US Patent No. 6,143,763; EP Patent No. 796248), pimobendan, amrinone, milrinone, vesnarinone (OPC-8212), lixazinone, trequinsin and cilostamide, among these cilostazol and K-134 are particularly preferable. These medical agents can be used as a salt or solvate as necessary. For PDE3 inhibitors of the present invention, one or more of agent(s) selected from a group consisting of PDE inhibitors of the above.

HMG-CoA reductase inhibitors for combined administration of the present invention is an HMG-CoA reductase inhibitor effective *in vivo*. Specific examples thereof are
lovastatin ((+)-(1S, 3R, 7S, 8S, 8aR)-1,2,3,7,8,8a-hexahydro-3,7-dimethyl-8-[2-[(2R,4R)-tetrahydro-4-hydroxy-6-oxo-2H-pyran-2-yl]ethyl]-1-naphthyl (S)-2-methylbutyrate (see US Patent No. 4,231,938);
simvastatin ((+)-(1S, 3R, 7S, 8S, 8aR)-1,2,3,7,8,8a-hexahydro-3,7-dimethyl-8-[2-[(2R,4R)-tetrahydro-4-hydroxy-6-oxo-2H-pyran-2-yl]ethyl]-1-naphtyl 2,2-dimethylbutanoate (see US Patent No. 4,444,784);
pravastatin ((+)-(3R,5R)-3,5-dihydroxy-7-[(1S, 2S, 6S, 8S, 8aR)- 6-hydroxy-2-methyl-8-[(S)-2-methylbutyryloxy]-1, 2, 6, 7, 8, 8a-hexahydro-1-naphtyl]heptanoic acid (see US Patent No. 4,346,227)
fluvastatin ((3RS, 5SR, 6E)-7-[3-(4-fluorophenyl)-1-(1-methylethyl)-1H-indol-2-yl]-3,5-dihydroxy-6-heptenoic acid (see US Patent No. 5,354,772);
atorvastatin ((3R, 5R)-7-[2-(4-fluorophenyl)-5-isopropyl-3-phenyl-4-phenylcarbamoyl-1H-pyrrol-1-yl]-3,5-dihydroxy heptanoic acid (see US Patent No. 5,273,995);
cerivastatin ((3R, 5S)-erythro-(E)-7-[4-(4-fluorophenyl)-2,6-diisopropyl-5-methoxymethyl-piridin-3-yl]-3,5-dihydroxy-6-heptenoic acid (see US Patent No. 5,177,080)
mevastatin ((+)-(1S, 3R, 7S, 8S, 8aR)-1,2,3,7,8,8a-hexahydro-7-methyl-8-[2-[(2R, 4R)-tetrahydro-4-hydroxy-6-oxo-2H-pyran-2-yl]ethyl]-1-naphthyl (S)-2-methyl-butyrate (see US Patent No. 3,983,140); rosuvastatin (7-[4-(4-fluorophenyl)-6-isopropyl-2-(N-methyl-N-methanesulfonylaminopyridin)-5-yl]-(3R, 5S)-dihydroxy-(E)-6-heptenoic acid (see US Patent No. 5,260,440, Japanese Patent No. 2648897);
pitavastatin ((3R, 5S, 6E)-7-[2-cycloropyl-4-(4-fluorophenyl)-3-quinolyl]-3,5-dihydroxy-6-heptenoic acid (see US Patent No. 5,856,336, Japan Patent No. 2569746)

HMG-CoA reductase inhibitors abovementioned can be used as salts or solvates thereof, if pharmaceutically required. Particularly, preferable inhibitor is pitavastatin. As HMG-CoA reductase inhibitors of the present invention, one or more of the agent(s) selected from the above specified group of HMG-CoA reductase inhibitors can be used.

HMG-CoA reductase inhibitors and PDE3 inhibitors of the present invention prepared individually according to the methods known in the art or those shown below can be administered in combination either concurrently or at an interval. The HMG-CoA reductase inhibitor and PDE3 inhibitor can also be formulated in an unitary dosage at the appropriate ratio according to each effective dose.

The agents can be prepared into an oral agent or parenteral agent, such as an oral agent, injection, suppository, ointment, and adhesive preparation comprising a pharmaceutically acceptable carrier in a suitable form for administration can be prepared according to the methods known in the art. An HMG-CoA reductase inhibitor and a PDE3 inhibitor of the present invention can be used as a pharmaceutically acceptable salt thereof, a hydrate thereof, or a hydrate of the pharmaceutically acceptable salt thereof, which can be obtained by the conventional methods. Examples of acids to form pharmaceutically acceptable salts, e.g. acid addition salts, include inorganic acids such as hydrochloric acid, sulfuric acid, nitric acid, phosphoric acid, hydrobromic acid, hydroiodic acid; and organic acids such as acetic acid, lactic acid, succinic acid, tartaric acid, malic acid, maleic acid, fumaric acid, citric acid, ascorbic acid, methanesulfonic acid, besilic acid, and toluenesulfonic acid. HMG-CoA reductase inhibitors and PDE3 inhibitors of the present invention can include the above described compounds as the active ingredient(s) either solely or in combination of two or more.

Solid preparations for oral use can be prepared by adding excipients, and as necessary binders, disintegrants, lubricants, colorants, flavouring substances, or flavouring agents to for example an HMG-CoA reductase inhibitor and a PDE3 inhibitor or pharmaceutically acceptable salt thereof or hydrate thereof. Then, according to the conventional methods, tablets, coated tablets, granulated agents, powdered medicines, and capsules can be prepared. Any of the additives generally used in the art can be used as such additives. Examples of excipients include lactose, sucrose, sodium chloride, glucose, starch, calcium carbonate, kaolin, microcrystalline cellulose and silic acid. Examples of binders include water, ethanol, propanol, simple syrup, dextrose in water, starch in water, gelatine in water, carboxymethyl cellulose, hydroxypropyl cellulose, hydroxypropyl starch, methyl cellulose, ethyl cellulose, shellack, calcium phosphate and polyvinylpyrrolidone. Examples of disintegrants include dry starch, sodium alginate, powdered agar, sodium hydrogen carbonate, calcium carbonate, sodium lauryl sulfate, stearic acid monoglyceride and lactose. Examples of lubricants include purified tarc, stearate, borax and polyethyleneglycol. Examples of flavoring substances include sucrose, wild orange peel, citric acid and tartaric acid.

Liquid preparations for oral use can be prepared by adding flavoring substances, buffer agents, stabilizing agents or flavoring agents as necessary, to for example an HMG-CoA reductase inhibitor and a PDE3 inhibitor or pharmaceutically acceptable salt thereof or hydrate thereof. According to the conventional methods, liquid for internal use, syrup and elixir can be prepared. Flavoring substances that can be used herein are described as above, an example of buffer agents includes sodium citrate, and examples of stabilizers include tragacanth, gum arabic, and gelatine.

Injections can be prepared by adding for example pH adjusting agents, buffer agents, stabilizers, tonicity agents, topical anesthetic agents or the like as necessary. According to the conventional methods, hypodermic, intramuscular and intravenous injections can be prepared. Examples of pH adjusting agents and buffer agent include sodium citrate, sodium acetate and sodium phosphate. Examples of stabilizers include sodium pyrosulphite, EDTA, thioglycolic acid, thiolactic acid. Examples of topical anesthetic agents include procaine hydrochloride and lidocaine. Examples of tonicity agents include sodium hydrochloride, and glucose. Other dosage forms can be prepared according to the known methods.

Also, pharmaceutical formulations prepared as described above can be packaged individually and used by removed from each package. Each pharmaceutical formulation can also be packaged in a suitable form for every combined administration.

A pharmaceutical composition obtained as the above that is effective in lowering triglyceride (TG) is useful not only in preventing and/or treating such as hypertriglyceridemia but also preventing and/or treating hypercholesterolaemia, arteriosclerosis, peripheral hypoperfusion, areterial/venous thrombosis, diabetes and its complication, hypertension and its complication, and metabolic disorder syndrome.

Dose of a pharmaceutical composition of the present invention having hypotriglyceride action varies from patients' body weight, age, sex, symptoms, administration routes and number of doses. Usually the composition is administered as HMG-CoA reductase inhibitor in amounts of 0.01-1000 mg preferably 0.1-100 mg, also administered as a PDE3 inhibitor in amounts of 0.01-5000 mg preferably 0.1-500 mg, orally/parenterally, once/in several times, in a single formulation/in combination with each. When pitavastatin is used as an HMG-CoA reductase inhibitor, it is preferable to be administered orally in an amount of 1-2 mg once a day after supper. When each of an HMG-CoA reductase inhibitor and a PDE3 inhibitor are in a single formulation, they can be administered either concurrently or separately at intervals of 15 minutes to 6 hours.

### EXAMPLES

Hereinafter, the present invention is described in more detail by reference to the Examples, but the technical scope of the present invention is not limited to the Examples.

### Example 1

Plasma TG lowering effect when pitavastatin calcium and K-134 are administered in combination

Plasma TG lowering effect when pitavastatin calcium was administered in combination with K-134 or cilostazol was measured according to the following method 1-4.

### 1. Test Animal and Rearing Environment

A 7-week-old Wistar rat (CLEA Japan, Inc.) was housed in an animal room where light/dark cycle (light period with room lighting from 7.00 to 19.00), temperature of 23 ± 3 °C, and humidity of 55 ± 15 % were maintained through the experimental period, and solid feed (CE2; Oriental Yeast Co., Ltd.) and tap water were consumed freely.

### 2. Medicinal Preparation

Pitavastatin calcium, K-134 and cilostazol were each suspended in aqueous solution (1.0 mass %) of hydroxypropyl methyl cellulose (Shin-Etsu Chemical Co. , Ltd.) and prepared in order that the administration dose was 1 mL/kg. The suspension was stored in a colored bottle and refrigerated at 4 °C. Preparation was performed every seven days.

### 3. Test Method

36 rats were divided into 6 administration groups (each group consisted of 6 samples): (1) control; (2) pitavastatin calcium (10mg/kg) alone; (3) K-134 (100mg/kg) alone; (4) cilostazol (100mg/kg) alone; (5) combination of pitavastatin calcium (10mg/kg) and K-134 (100mg/kg); and (6) combination of pitavastatin calcium (10mg/kg) and cilostazol(100mg/kg).
Pitavastatin was orally administered once daily at 4.00 pm for 14 days repeatedly, and K-134 or cilostazol was orally administered twice daily at 9.00 am and 4.00 pm for 14 days repeatedly. To the control group sodium hydroxypropyl methylcellulose (1.0 mass %, 1mL/kg) in aqueous solution was orally administered once a day at 4.00 pm. Blood sample was taken from each group after 22-hours fasting following the final administration in the afternoon, and plasma TG was measured.

### 4. Data Processing Method

The results were shown with the average i standard deviation. Figure 1 shows the test result.

As shown in figure 1 (pitavastatin calcium is referred to as pitavastatin), plasma TG level was almost unchanged by a single administration of each agent; however, plasma TG level was significantly lowered when pitavastatin was administered in combination with K-134 or cilostazol. Accordingly, a combined administration of pitavastatin and K-134 or cilostazol showed excellent hypotriglyceridemic effect in plasma compared to a single administration of each.

### Industrial Applicability

The present invention relates to a significant hypotriglyceridemic effect by administering an HMG-CoA reductase inhibitor and a PDE3 inhibitor in combination. The present invention also showed that combination administration of HMG-CoA reductase inhibitor and Hyprotriglyceridemic agent had sufficient effect in lowering triglyceride without producing serious side effects such as high incidence of rhabdomyolysis, unlike combination administration of existing HMG-CoA reductase inhibitors and the fibrates. The agent of the present invention is remarkably useful in preventing/treating varieties of hypertriglyceridemia particularly hypertriglyceridemia with hyperlipidemia.

## Claims

1. Use of an HMG-CoA reductase inhibitor and a PDE3 inhibitor in preparing a pharmaceutical composition comprising effective amounts of an HMG-CoA reductase inhibitor and a PDE3 inhibitor for lowering triglyceride (TG), wherein the HMG-CoA reductase inhibitor is one or more than two medical agents selected from the group consisting of pravastatin, simvastatin, fluvastatin, cerivastatin, atorvastatin, rosuvastatin and pitavastatin.

2. Use according to claim 1, wherein the PDE3 inhibitor is cilostazol.

3. Use according to claim 1, wherein the PDE3 inhibitor is K-134((-)-6-[3[3-cyclopropyl-3-[(1R,2R)-2-hydroxycyclohexyl]ureido]-propoxy] -2-(1H)-quinoline).

4. Use according to claim 1, wherein the HMG-CoA reductase inhibitor is one or more than two medical agent selected from a group consisting of atorvastatin, rosuvastatin and pitavastatin.

5. Use according to claim 4, wherein the HMG-CoA reductase inhibitor is pitavastatin.

6. A pharmaceutical composition for use in a method of lowering triglyceride (TG) comprising an HMG-CoA reductase inhibitor and a PDE3 inhibitor, and pharmaceutically acceptable carrier, wherein the HMG-CoA reductase inhibitor is one or more than two medical agents selected from the group consisting of pravastatin, simvastatin, fluvastatin, cerivastatin, atorvastatin, rosuvastatin and pitavastatin.

## Patentansprüche

1. Verwendung eines HMG-CoA-Reduktase-Inhibitors und eines PDE3-Inhibitors bei der Herstellung einer pharmazeutischen Zusammensetzung, die wirksame Mengen eines HMG-CoA-Reduktase-Inhibitors und eines PDE3-Inhibitors umfasst, zur Senkung des Triglycerids (TG), wobei der HMG-CoA-Reduktase-Inhibitor ein medizinisches Mittel oder mehr als zwei medizinische Mittel ist, das/die aus der Gruppe, bestehend aus Pravastatin, Simvastatin, Fluvastatin, Cerivastatin, Atorvastatin, Rosuvastatin und Pitavastatin, ausgewählt ist/sind.

2. Verwendung gemäß Anspruch 1, wobei der PDE3-Inhibitor Cilostazol ist.

3. Verwendung gemäß Anspruch 1, wobei der PDE3-Inhibitor K-134((-)-6-[3[3-Cyclopropyl-3-[(1R,2R)-2-hydroxycyclohexyl]ureido]-propoxy]-2-(1H)-chinolin) ist.

4. Verwendung gemäß Anspruch 1, wobei der HMG-CoA-Reduktase-Inhibitor ein medizinisches Mittel oder mehr als zwei medizinische Mittel ist, das/die aus einer Gruppe, bestehend aus Atorvastatin, Rosuvastatin und Pitavastatin, ausgewählt ist/sind.

5. Verwendung gemäß Anspruch 4, wobei der HMG-CoA-Reduktase-Inhibitor Pitavastatin ist.

6. Pharmazeutische Zusammensetzung zur Verwendung in einem Verfahren zur Senkung von Triglycerid (TG), die einen HMG-CoA-Reduktase-Inhibitor und einen PDE3-Inhibitor und einen pharmazeutisch verträglichen Träger umfasst, wobei der HMG-CoA-Reduktase-Inhibitor ein medizinisches Mittel oder mehr als zwei medizinische Mittel ist, das/die aus der Gruppe, bestehend aus Pravastatin, Simvastatin, Fluvastatin, Cerivastatin, Atorvastatin, Rosuvastatin und Pitavastatin, ausgewählt ist/sind.

## Revendications

1. Utilisation d'un inhibiteur de la HMG-CoA réductase et d'un inhibiteur de la PDE3 dans la préparation d'une composition pharmaceutique comprenant des quantités efficaces d'un inhibiteur de la HMG-CoA réductase et d'un inhibiteur de la PDE3 destinée à baisser les taux de triglycérides (TG), dans laquelle l'inhibiteur de la HMG-CoA réductase est un ou plus de deux agents médicaux choisis dans le groupe comprenant la pravastatine, la simvastatine, la fluvastatine, la cérivastatine, l'atorvastatine, la rosuvastatine et la pitavastatine.

2. Utilisation selon la revendication 1, dans laquelle l'inhibiteur de la PDE3 est le cilostazol.

3. Utilisation selon la revendication 1, dans laquelle l'inhibiteur de la PDE3 est la K-134((-)-6-[3[3-cyclopropyl-3-[(1R,2R)-2-hydroxycyclo-hexyl]uréido] -propoxy]-2-(1H)-quinoline).

4. Utilisation selon la revendication 1, dans laquelle l'inhibiteur de la HMG-CoA réductase est un ou plus de deux agents médicaux choisis dans un groupe comprenant l'atorvastatine, la rosuvastatine et la pitavastatine.

5. Utilisation selon la revendication 4, dans laquelle l'inhibiteur de la HMG-CoA réductase est la pitavastatine.

6. Composition pharmaceutique à utiliser dans un procédé destiné à baisser les taux de triglycérides (TG) comprenant un inhibiteur de la HMG-CoA réductase et un inhibiteur de la PDE3, et un transporteur pharmaceutiquement acceptable, dans laquelle l'inhibiteur de la HMG-CoA réductase est un ou plus de deux agents médicaux choisis dans le groupe comprenant la pravastatine, la simvastatine, la fluvastatine, la cérivastatine, l'atorvastatine, la rosuvastatine et la pitavastatine.
